# FASCICULE DE BREVET EUROPEEN

(11) **EP 1 377 271 B1**
(45) Date de publication et mention de la délivrance du brevet: **01.10.2008**
(21) Numéro de dépôt: 02727650.0
(22) Date de dépôt: 03.04.2002
(51) Int. Cl.: A61K 8/06, A61K 8/37, A61Q 19/00

(54) **EMULSION A BASE D'ALCOOLS GRAS ET DE GLUCOLIPIDES A STABILITE AMELIOREE ET VISCOSITE ELEVEE UTILISABLE EN COSMETIQUE.**
EMULSION AUF DER BASIS VON FETTALKOHOLEN UND GLUCOLIPIDEN MIT VERBESSERTER STABILITÄT UND HOCHVISKOSE SOWIE IHRE VERWENDUNG IN KOSMETIK
EMULSION BASED ON FATTY ACID AND GLUCOLIPID WITH IMPROVED STABILITY AND HIGHER VISCOSITY for USE IN COSMETICS

(30) Priorité: 11.04.2001 FR 0104980
(43) Date de publication de la demande: 07.01.2004
(73) Titulaire: Laboratoire Nuxe, 75010 Paris (FR)
(72) Inventeur: LECLERE, Jacques, F-45500 SAINT-GONDON (FR); LECONTE, Nadine, F-75011 PARIS (FR)
(74) Mandataire: L'Helgoualch, Jean
(86) Numéro de dépôt international: PCT/FR2002/001158
(87) Numéro de publication internationale: WO 2002/083094

(56) Documents cités:
- EP-A- 0 628 305
- EP-A- 0 629 396
- EP-A- 0 834 306
- EP-A- 1 000 542
- FR-A- 2 720 934
- FR-A- 2 790 977

## Description

La présente invention concerne une émulsion à stabilité améliorée, et plus particulièrement une émulsion à viscosité relativement élevée, présentant une stabilité améliorée, utilisable dans des compositions cosmétiques et dermatologiques, ainsi qu'un procédé de préparation.

Dans l'industrie des produits cosmétiques et pharmaceutiques susceptibles d'être présentés sous forme de crèmes, pommades, laits et émulsions diverses, de type huile-dans-eau (H/E) ou eau-dans-huile (E/H), on utilise de nombreux agents émulsionnants qui sont généralement des agents tensioactifs, ioniques ou non ioniques, hydrophiles ou lipophiles, et par exemple des émulsionnants polyoxyéthylés. Ces derniers présentent toutefois des inconvénients liés à leur toxicité vis-à-vis des cellules cutanées et à leur pouvoir irritant pour la peau.

On utilise souvent des émulsionnants choisis parmi les sucro-esters ou glucolipides du type oléyl-glucoside, myristyl glucoside, lauroyl glucoside, stéaryl glucoside, isostéaryl glucoside ou encore béhényl glucoside, qui peuvent être associés à des alcools gras dans un rapport en poids compris entre 10 à 20 % de glucolipide et 80 à 90 % d'alcool gras. Les alcools gras peuvent être choisi par exemple parmi les alcools oléylique, myristylique, arachidylique, stéarylique, isostéarylique, cétylique, béhénylique, etc.

Notamment dans le commerce, on connaît des compositions auto-émulsionnables telles que le Montanov 68^{®} de la société Seppic, associant un alcool cétylstéarylique en C₁₆-C₁₈ et un cétéarylglucoside, ou le Montanov 202^{®} associant un alcool arachidylique, un alcool béhénylique et un arachidyl glucoside, de la société Seppic. Ces compositions auto-émulsionnables présentent cependant une viscosité insuffisante.

Le brevet EP-A-553.241 décrit également des associations d'alcools gras et de glucolipides susceptibles de remplacer les émulsionnants polyoxyéthylés, mais les émulsions obtenues présentent une viscosité insuffisante pour certaines applications où des émulsions épaisses sont recherchées.

Une solution connue dans l'état de la technique pour augmenter la viscosité de l'émulsion consiste à introduire des agents viscosants supplémentaires, mais cette addition a généralement des effets néfastes sur leur stabilité. Ainsi, l'addition de cire, en tant qu'agent viscosant, augmente de manière certaine la viscosité de la composition, et des cires de riz, ou de sumac, ou de candellila peuvent par exemple être utilisées, mais la composition a alors un toucher trop sec, pâteux, une couleur inappropriée, et ces inconvénients sont définitivement rédhibitoires pour l'utilisateur.

D'une manière générale, la mise au point d'émulsions à viscosité relativement élevée, correspondant à la viscosité des crèmes épaisses et des crème souples, à la différence des laits fluides, se heurte souvent à des difficultés résultant de la dégradation de la stabilité sous l'influence des agents viscosants ajoutés pour augmenter la viscosité de la composition. Ainsi l'utilisation de polymères viscosants du type Carbopol^{®} a pour inconvénient leur incompatibilité avec les structures réticulées des glucolipides. Il en est de même avec les gommes xanthane. En effet, il y a une concurrence entre les structures réticulées de l'agent viscosant et celles des glucolipides. L'émulsion est donc déstabilisée, car les structures réticulées des glucolipides ne sont pas toutes incluses dans des structures micellaires.

Si certains gélifiants tels que le Sepigel^{®} de la société Seppic parviennent à stabiliser de telles compositions, ils ne permettent pas cependant d'en augmenter la viscosité.

Le brevet EP-A-629.396 propose d'ajouter un co-émulsionnant à des compositions du type huile végétale associée avec une composition autoémulsionnable d'alcool gras et d'alkylpolyosides. L'addition du co-émulsionnant permet de former une émulsion huile-dans-eau comprenant une phase huileuse contenant une huile végétale ayant plus de 40% de triglycérides d'acide linoléique. Le co-émulsionnant est choisi parmi les alcools gras et les acides gras ayant 14 à 22 atomes de carbone. Il s'agit notamment d'un acyl glutamate, mais ce type de coémulsionnant concourt à des émulsions ou des crèmes fluides et ne convient donc pas à des émulsions et crèmes épaisses à viscosité relativement élevée. Le brevet EP 628305 décrit une émulsion huile-dans-eau associant a) une huile végétale à base de triglycérides d'acide linoléique, b) une composition auto-émulsionnable d'alcool gras, d'alkylpolyoside et de polyoside, c) un alcool gras saturé ou un acide gras saturé constituant un co-émulsionnant, et d) un gélifiant. L'utilisation du gélifiant permet de stabilise l'émulsion, dont la viscosité, cependant, reste peu élevée.

On connaît également, du document FR 2 790 977, des émulsions eau dans huile contenant une composition auto-émulsionnable à base d'alcools gras et de glucolipides, ainsi que de la cire d'abeille en tant qu'agent stabilisant. Ces produits ne présentent toutefois pas les propriétés de viscosité recherchées.

Il existe donc un besoin non totalement satisfait d'émulsions épaisses à viscosité élevée et à bonne stabilité.

Or, les travaux réalisés par la demanderesse ont montré de manière inattendue qu'il est possible d'obtenir des émulsions épaisses à viscosité élevée et à bonne stabilité au moyen d'agents susceptibles de générer une augmentation de la viscosité de l'émulsion par une élévation de la viscosité de la phase grasse.

La présente invention a donc pour objet une émulsion à viscosité relativement élevée et à stabilité améliorée.

L'invention a également pour objet une émulsion à viscosité relativement élevée et à stabilité améliorée utilisable en cosmétique qui présente également une texture fine.

L'invention a aussi pour objet un procédé de préparation d'une émulsion utilisable dans des compositions cosmétiques et dermatologiques, permettant d'obtenir dans de bonnes conditions des émulsions à la fois épaisses et stables.

L'émulsion suivant la présente invention est une émulsion huile-dans-eau, à base d'association auto-émulsionnable d'alcools gras et de glucolipides dans un rapport en poids compris entre 10:1 et 4:1, et elle se distingue en ce qu'elle comprend en outre un agent viscosant de la phase grasse et un acétylglycéride.

L'agent viscosant de la phase grasse est un triglycéride choisi parmi la tri-oléine, la trilaurine, la tristéarine, et la tri-isostéarine. De préférence l'agent viscosant est la tristéarine.

L'émulsion suivant la présente invention comprend un co-émulsionnant. Ce co-émulsionnant est un acétylglycéride, par exemple un glycérylstéarate d'acétyle. On a observé que cet acétylglycéride permet d'améliorer l'étalement des huiles (film monomoléculaire) et de procurer un effet "colloïde protecteur" dans l'émulsion. Dans ce cas, l'émulsion obtenue est plus fine, tout en présentant une forte viscosité et une grande stabilité.

Par exemple, on peut incorporer une proportion d'UNITWIX^{®} de la société Guardian Laboratories dans la composition. Le produit UNITWIX^{®} comprend un mélange de tristéarine et de stéarate d'acétylglycéryle. Le produit UNITWIX^{®} est couramment utilisé comme facteur de consistance, mais il est ici avantageusement incorporé à la composition selon l'invention en tant qu'agent co-émulsionnant. Il coopère notamment avec les dérivés glucosidiques prévus dans la composition. La proportion d'UNITWIX^{®} est avantageusement comprise entre 0,5 et 3 % en poids environ par rapport au poids total de l'émulsion. D'une manière générale, la proportion d'acétylglycéride est inférieure à 5% en poids par rapport au poids total de l'émulsion.

Dans un mode de réalisation préféré, la phase grasse comporte des lipoglucosides, par exemple un mélange de Montanov68^{®} et/ou de Montanov202^{®}. Une proportion de Montanov68^{®} est par exemple inférieure à 10% en poids par rapport au poids total de l'émulsion, de préférence entre 0,5 et 5%. Une proportion de Montanov202^{®} est par exemple inférieure à 5% en poids par rapport au poids total de l'émulsion, de préférence comprise entre 1 et 4,5%.

De préférence, on prépare cette émulsion de manière à obtenir une émulsion lamellaire. Une émulsion lamellaire se
caractérise notamment par la présence de glucolipides arrangés de manière à former des micelles présentant une bicouche lipidique. La présence de Montanov202® dans la composition permet avantageusement d'obtenir une telle structure.

Suivant une forme préférentielle de réalisation de l'invention, la quantité d'alcool gras de l'émulsion est inférieure à 10%, et avantageusement inférieure à 6% en poids par rapport au poids total de l'émulsion. La phase grasse comporte des composants classiques couramment utilisés dans les techniques des compositions cosmétiques et dermatologiques. Notamment, la phase grasse comporte des alcools gras, des lipoglucides, des esters d'alcool gras, des silicones, des huiles végétales, de l'huile de vaseline, ou encore un perhydrosqualène.

Dans une première variante, la composition comporte 5% de Montanov68® et entre 0,5% et 3% d'UNITWIX®, les pourcentages étant exprimés en poids par rapport au poids total de l'émulsion.

Dans une deuxième variante, la composition comporte entre 0,5% et 4% de Montanov68®, entre 1% et 4% de Montanov202®, de telle sorte que le pourcentage de l'ensemble des produits Montanov® représente 5%, et entre 0,5% et 3% d'UNITWIX®, les pourcentages étant exprimés en poids par rapport au poids total de l'émulsion.

Pour stabiliser une telle émulsion, la composition comprend des agents stabilisants, par exemple dans une proportion inférieure à 1% en poids par rapport au poids total de l'émulsion. L'agent stabilisant est un agent du type Sepigel®, de la société Seppic, dans une proportion comprise entre 0,6% et 1%. Par exemple, il comprend une proportion de Sepige1305® et/ou une proportion de Simulge1502®.

Le procédé de préparation d'une émulsion à viscosité élevée et à stabilité améliorée suivant l'invention consiste à chauffer séparément la phase grasse et la phase aqueuse. On amène chacune de ces phases à des températures par exemple comprises entre 75 et 80°C. On les mélange à chaud en versant de préférence la phase grasse dans la phase aqueuse. On agite le mélange ainsi obtenu au moyen d'une turbine pour l'homogénéiser. Un mélangeur utilisé peut comporter un triple mouvement permettant ainsi de créer des mouvements rapides parallèlement à des mouvements lents. On mélange par exemple pendant une dizaine de minutes avec une vitesse de rotation de la turbine de l'ordre de 3000 tours par minute.

Ensuite, le mélange est refroidi lentement et progressivement sous agitation lente. Lorsque le mélange atteint une température de préférence comprise entre 60 et 62°C, on ajoute l'agent stabilisant. On continue l'homogénéisation du mélange en maintenant par exemple la vitesse de rotation de la turbine à une valeur de l'ordre de 3000 tours par minute.

Au cours du refroidissement, on ajoute, par exemple des principes actifs et du parfum, lorsque la température du mélange atteint une température de l'ordre de 40°C. Ensuite, le mélange est amené lentement à une température de 28 à 30°C, à laquelle il peut être stocké, et/ou conditionné.

De préférence, on introduit le triglycéride, et éventuellement l'acétylglycéride, dans la phase grasse à une température inférieure à la température d'inversion de phase de l'émulsion.

Lorsque cette température est trop élevée, on observe une inversion de phase dans l'émulsion qui se traduit par une perte de stabilité. A l'inverse, une température trop faible provoque une déstabilisation par cristallisation.

La composition suivant la présente invention peut en outre contenir divers adjuvants et excipients couramment utilisés dans les techniques des compositions cosmétiques et dermatologiques. Elle peut, par exemple, contenir des conservateurs, des épaississants, des gélifiants, des agents émulsionnants complémentaires, des agents humectants, des antioxydants, des agents hydratants, des tensioactifs, des parfums, et divers additifs destinés à améliorer les propriétés physiques de la composition.

Il peut aussi être avantageux d'incorporer dans la composition un actif complémentaire destiné à améliorer le comportement de la peau, tel que le tocophérol. Le tocophérol, ou un dérivé tel que l'acétate de tocophérol, est tout particulièrement utile en raison de sa capacité à piéger les radicaux libres et à empêcher l'oxydation des corps gras de l'émulsion.

Les épaississants peuvent par exemple être choisis parmi les dérivés acryliques, de préférence ceux ayant un réseau spatial différent de celui des glucolipides, et à condition de réaliser un refroidissement rapide de la composition après mélange à chaud des phases grasses et aqueuses. De préférence, un gélifiant tel que l'amidon, ou des dérivés de l'amidon peuvent être avantageusement incorporés pour améliorer l'onctuosité d'une telle composition.

L'agent hydratant ou humectant peut être choisi parmi un polyol, le sorbitol, le maltitol, le pentaérythritol, la glycérine, le glycol, le propylglycol, le phenoxyethanol, les polyacrylates et polyméthacrylates de glycéryle, le glycérol ou des dérivés du glycérol, l'acide hyaluronique ou des dérivés de cet acide. D'une manière générale, tout agent hydratant convenant aux compositions cosmétiques ou dermatologiques peut être utilisé dans la présente invention. L'agent humectant est avantageusement introduit dans la phase aqueuse lors de la préparation de l'émulsion.

Les conservateurs usuels de la technique des compositions cosmétiques ou dermatologiques peuvent être utilisés dans l'invention, et par exemple l'acide benzoïque et des parahydroxybenzoates de méthyle et/ou de propyle, isolément ou en combinaison.

Des agents de protection contre les rayons ultra-violets peuvent également être utilisés. Ces agents de protection peuvent être par exemple des filtres solaires UV-A et UV-B tels que la benzophénone ou un dérivé de benzophénone, ou un ester d'acide cinnamique et plus particulièrement le méthoxycinnamate d'octyle (Parsol MCX®), le méthoxycinnamate d'éthylhexyle, le 4-isopropyl dibenzoylméthane (Eusolex 8020) et le butylméthoxy dibenzoylméthane (Parsol 1789^{®}). On peut aussi utiliser des pigments formant un écran anti-ultraviolet, qui peuvent par exemple être choisis parmi les oxydes de titane, de zinc, de fer ou d'aluminium, ou encore des colorants de fonds de teint tels que kaolin, talc et laques.

Des agents humectants peuvent également être utilisés, ils peuvent être choisis parmi la glycérine, le propylène glycol et le polyéthylène glycol.

Les compositions peuvent se présenter sous forme de crèmes, émulsions huile-dans-eau, masques, sticks ou pommades. Un mode de réalisation préféré de l'invention se présente sous forme de crème régénératrice pour des peaux matures, ou des peaux nécessitant un soin complémentaire à appliquer de préférence le soir, pour une action pendant le sommeil.

Les caractéristiques et avantages de la présente invention apparaîtront plus en détail dans la description qui suit, relative à des formes préférentielles de réalisation. Dans ces exemples, les parties et pourcentages sont exprimés en poids, sauf indication contraire.

### Exemple 1

On prépare une crème régénératrice "Nuit" en opérant comme indiqué ci dessous, en utilisant les techniques usuelles de l'industrie des produits cosmétiques et dermatologiques. La composition pondérale est la suivante.

| Phase aqueuse | |
|---|---|
| Eau déminéralisée | q.s.p. 100,0 g |
| Conservateur | 0,5 g |
| Miel | 3,0 g |

| Phase grasse | |
|---|---|
| Alcool stéarylique | 1,0 g |
| Stéarate de glycérol | 0,5 g |
| Montanov 68® | 3,5 g |
| Montanov 202® | 1,5 g |
| Citrate tricaprylique | 2,0 g |
| Maléate dicaprylique | 2,5 g |
| Octanoate cétéarylique | 2,0 g |
| Diméthylpolysiloxane | 1,0 g |
| Dioctyl succinate | 5,0 g |
| UNITWIX | 2,5 g |
| Huile végétale | 4,5 g |
| Tocophérol | 0,7 g |
| Phase C | |
| Sepige1305® / Simulge1502® | 1,0 g |
| Phase D | |
| Extrait de levure | 6,0 g |
| Parfum | 0,5 g |

Notamment la phase grasse et la phase aqueuse sont préparées et mélangées comme indiqué ci-dessus. La phase C est ajouté dans le mélange préliminaire lorsque celui ci est descendu à des températures de l'ordre de 60°C. Enfin, la phase D est incorporée lorsque le mélange homogénéisé est refroidi à une température de l'ordre de 40°C.

### Exemple 2

On prépare une crème "pour peaux matures" en opérant comme indiqué ci dessus, en utilisant les techniques usuelles de l'industrie des produits cosmétiques et dermatologiques. La composition pondérale est la suivante.

| Phase aqueuse | |
|---|---|
| Eau déminéralisée | q.s.p. 100,0 g |
| Conservateur | 0,5 g |
| Extrait de Calendula | 3,0 g |

| Phase grasse | |
|---|---|
| Alcool stéarylique | 1,2 g |
| Stéarate de glycérol | 0,7 g |
| Montanov 68® | 4,8 g |
| Citrate tricaprylique | 2,0 g |
| Maleate dicaprylique | 2,5 g |
| Octanoate cétéarylique | 2,0 g |
| Phényl trimethicone | 1,0 g |
| Dioctyl succinate | 3,5 g |
| UNITWIX | 2,0 g |
| Huile de vaseline | 6,0 g |
| Tocophérol | 0,7 g |

| Phase C | |
|---|---|
| Sepigel305® / Simulge1502® | 1,0 g |

| Phase D | |
|---|---|
| Phytostérol de soja | 0,8 g |
| Parfum | 0,5 g |

Les différentes phases sont préparées et mélangées comme indiqué dans l'exemple 1.

## Revendications

1. Emulsion à base d'association auto-émulsionnable d'alcools gras et de glucolipides dans un rapport en poids compris entre 10:1 et 4:1, **caractérisée en ce qu'**elle comprend en outre un agent viscosant de la phase grasse et un acétylglycéride, ledit agent viscosant étant un triglycéride choisi parmi la trioléine, la trilaurine, la tristéarine, la tri-isostéarine.

2. Emulsion selon la revendication 1, **caractérisée en ce que** l'agent viscosant choisi est la tristéarine.

3. Emulsion selon l'une quelconque des revendications précédentes, **caractérisée en ce qu'**une proportion d'agent viscosant est comprise entre 0,5 et 10% en poids par rapport au poids total de l'émulsion.

4. Emulsion selon l'une quelconque des revendications précédentes, **caractérisée en ce que** l'acétylglycéride est le glycérylstéarate d'acétyle.

5. Emulsion selon l'une quelconque des revendications précédentes, **caractérisée en ce que** la proportion d'acétylglycéride est inférieure à 5% en poids par rapport au poids total de l'émulsion.

6. Emulsion selon la revendication 1, **caractérisée en ce qu'**elle comprend un mélange de tristéarine et de glycérylstéarate d'acétyle dans une proportion comprise entre 0,5 et 3% en poids par rapport au poids total de l'émulsion.

7. Procédé de préparation d'une émulsion selon l'une quelconque des revendications précédentes, **caractérisé en ce que**
- on chauffe séparément la phase grasse et la phase aqueuse,
- on mélange ces deux phases en les homogénéisant, et au cours du refroidissement,
- on ajoute successivement les agents stabilisants, puis les autres additifs.

8. Procédé selon la revendication 7, **caractérisé en ce qu'**on introduit le triglycéride dans la phase grasse à une température inférieure à la température d'inversion de phase de l'émulsion.

9. Procédé selon la revendication 8, **caractérisé en ce que** l'acétylglycéride est introduit dans la phase grasse.

10. Composition cosmétique, **caractérisée en ce qu'**elle comprend une émulsion selon l'une quelconque des revendications 1 à 6.

## Claims

1. Emulsion based on a self-emulsifying combination of fatty alcohols and glucolipids in a weight ratio from 10:1 to 4:1, **characterized in that** it further comprises a viscosing agent of the fatty phase and an acetylglyceride, said viscosing agent being selected from trioleine, trilaurine, tristearine, tri-isostearine.

2. Emulsion according to claim 1, **characterized in that** the selected viscosing agent is tristearine.

3. Emulsion according to any of the preceding claims, **characterized in that** a ratio of the viscosing agent is from 0.5 to 10% by weight of the total weight of the emulsion.

4. Emulsion according to any of the preceding claims, **characterized in that** the acetylglyceride is acetyl glycerylstearate.

5. Emulsion according to any of the preceding claims, **characterized in that** the ratio of acetylglyceride is lower than 5% by weight of the total weight of the emulsion.

6. Emulsion according to claim 1, **characterized in that** it comprises a mixture of tristearine and acetyl glycerylstearate in a weight ratio from 0.5 to 3% by weight of the total weight of the emulsion.

7. Method of preparation of an emulsion according to any of the preceding claims, **characterized in that**
- the fatty phase and the aqueous phase are heated separately,
- said two phases are mixed together and homogenised, and, during cooling,
- the stabilizing agents and then the other additives are successively added.

8. Method according to claim 7, **characterized in that** the triglyceride is introduced into the fatty phase at a temperature lower than the phase inversion temperature of the emulsion.

9. Method according to claim 8, **characterized in that** the acetylglyceride is introduced into the fatty phase.

10. Cosmetic composition, **characterized in that** it comprises an emulsion according to any of claims 1 to 6.

## Patentansprüche

1. Emulsion, basierend auf einer selbstemulgierbaren Verbindung von Fettalkoholen und Glucolipiden in einem Gewichtsverhältnis zwischen 10:1 und 4:1, **dadurch gekennzeichnet, dass** sie weiters ein viskositätssteigerndes Mittel der Fettphase und ein Acetylglycerid umfasst, wobei das viskositätssteigernde Mittel ein Triglycerid ist, das aus Triolein, Trilaurin, Tristearin und Triisostearin ausgewählt ist.

2. Emulsion nach Anspruch 1, **dadurch gekennzeichnet, dass** das ausgewählte viskositätssteigernde Mittel Tristearin ist.

3. Emulsion nach einem der vorangegangenen Ansprüche, **dadurch gekennzeichnet, dass** der Anteil des viskositätssteigernden Mittels, bezogen auf das Gesamtgewicht der Emulsion, zwischen 0,5 und 10 Gew.-% liegt.

4. Emulsion nach einem der vorangegangenen Ansprüche, **dadurch gekennzeichnet, dass** das Acetylglycerid Acetylglycerylstearat ist.

5. Emulsion nach einem der vorangegangenen Ansprüche, **dadurch gekennzeichnet, dass** der Anteil an Acetylglycerid, bezogen auf das Gesamtgewicht der Emulsion, weniger als 5 Gew.-% beträgt.

6. Emulsion nach Anspruch 1, **dadurch gekennzeichnet, dass** sie ein Gemisch aus Tristearin und Acetylglycerylstearat in einem Anteil, bezogen auf das Gesamtgewicht der Emulsion, zwischen 0,5 und 3 Gew.-% umfasst.

7. Verfahren zur Herstellung einer Emulsion nach einem der vorangegangenen Ansprüche, **dadurch gekennzeichnet, dass**:
- die Fettphase und die Wasserphase getrennt voneinander erhitzt werden,
- diese beiden Phasen durch Homogenisieren während des Abkühlens vermischt werden,
- nacheinander die Stabilisatoren und dann die anderen Additive zugesetzt werden.

8. Verfahren nach Anspruch 7, **dadurch gekennzeichnet, dass** das Triglycerid bei einer Temperatur, die niedriger als die Phaseninversionstemperatur der Emulsion ist, in die Fettphase eingeführt wird.

9. Verfahren nach Anspruch 8, **dadurch gekennzeichnet, dass** das Acetylglycerid in die Fettphase eingeführt wird.

10. Kosmetische Zusammensetzung, **dadurch gekennzeichnet, dass** sie eine Emulsion nach einem der Ansprüche 1 bis 6 umfasst.
